# EUROPÄISCHE PATENTSCHRIFT

(11) **EP 3 821 200 B1**
(45) Veröffentlichungstag und Bekanntmachung des Hinweises auf die Patenterteilung: **07.09.2022**
(21) Anmeldenummer: 19737038.0
(22) Anmeldetag: 02.07.2019
(51) Int. Cl.: G01B 9/02015, A61B 3/10, G01B 9/02091

(54) **OCT-SYSTEM UND OCT-VERFAHREN**
OCT SYSTEM AND OCT METHOD
SYSTÈME OCT ET PROCÉDÉ OCT

(30) Priorität: 11.07.2018 EP 18182845
(43) Veröffentlichungstag der Anmeldung: 19.05.2021
(73) Patentinhaber: HAAG-STREIT AG, CH-3098 Köniz (CH)
(72) Erfinder: ROBLEDO, Lucio, 3013 BERN (CH); STALDER, Peter, 3504 NIEDERHÜNIGEN (CH); HUBER-MEZNARIC, André, 3098 KÖNIZ (CH)
(74) Vertreter: Glawe, Delfs, Moll
(86) Internationale Anmeldenummer: PCT/EP2019/067676
(87) Internationale Veröffentlichungsnummer: WO 2020/011593

(56) Entgegenhaltungen:
- EP-A1- 2 564 767
- EP-A2- 2 719 324
- WO-A1-2016/196463
- SHANHUI FAN ET AL: "Dual band dual focus optical coherence tomography for imaging the whole eye segment", BIOMEDICAL OPTICS EXPRESS, Bd. 6, Nr. 7, 1. Juli 2015 (2015-07-01), Seite 2481, XP055501126, United States ISSN: 2156-7085, DOI: 10.1364/BOE.6.002481

## Beschreibung

Die Erfindung betrifft ein OCT-System mit einer OCT-Lichtquelle zum Aussenden von OCT-Licht in einen Objektstrahlengang und einen Referenzstrahlengang. Mit einem Detektor wird ein aus dem Objektstrahlengang und dem Referenzstrahlengang erzeugtes Interferenzsignal aufgenommen. Die Erfindung betrifft außerdem ein OCT-Verfahren.

Bei der optischen Kohärenztomographie (OCT) handelt es sich um ein bildgebendes Messverfahren. OCT-Licht wird auf ein Objekt, insbesondere menschliches Gewebe geleitet. Aus den zurückgeworfenen Anteilen des Lichts wird auf Streuzentren in dem Objekt geschlossen. Dazu wird der von dem Objekt zurückgeworfene Objektstrahlengang mit einem Referenz-Strahlengang überlagert. Die Bildinformation wird durch Auswerten des Interferenzsignals der beiden Strahlengänge gewonnen.

Bei OCT-Messungen ist die axiale Messtiefe allgemein begrenzt. Das Interferenzsignal hat die größte Signalstärke, wenn die optische Weglänge im Referenzstrahlengang und die optische Weglänge im Objektstrahlengang die gleiche Länge haben. Ein Punkt im Objektbereich, für den dies der Fall ist, wird als Referenzpunkt der OCT-Messung bezeichnet. Mit Fourier-Domain-OCT (FD-OCT) kann man auch Streuzentren erkennen, die vom Referenzpunkt beabstandet sind. Je größer der axiale Abstand zwischen einem Objektpunkt und dem Referenzpunkt ist, desto schwächer ist dabei aber das Interferenzsignal und desto schlechter ist die Qualität einer Bildinformation, die aus dem Interferenzsignal abgeleitet wird.

Wird ein OCT-Gerät für ophthalmologische Messungen verwendet, so sind häufig ein vorderer Augenabschnitt und ein hinterer Augenabschnitt von Interesse. Der vordere Augenabschnitt umfasst die Hornhaut (Cornea) und die Augenlinse. Der hintere Augenabschnitt umfasst die Netzhaut (Retina). Wird eine Messung vorgenommen, die sich über den vorderen Augenabschnitt und den hinteren Augenabschnitt erstreckt, so betrifft ein wesentlicher Teil der Bilddaten einen zwischen dem vorderen Augenabschnitt und dem hinteren Augenabschnitt liegenden Bereich. Dieser Teil der Bilddaten hat keinen relevanten Informationsgehalt.

Bekannt ist, zwei separate OCT-Interferometer vorzusehen, wobei ein Interferometer für den vorderen Augenabschnitt und ein Interferometer für den hinteren Augenabschnitt verwendet wird, EP 2 719 324 A2. Diese Lösung ist aufwendig, weil die wesentlichen Komponenten des OCT-Systems zweimal vorhanden sein müssen. Dies gilt auch für Lösungen, bei denen mit separaten Lichtquellen und separaten Detektoreinheiten für jeden der Messbereiche gearbeitet wird, WO 2001/038820 A1. Bekannt ist auch ein OCT-System, bei dem mechanisch zwischen einem Pfad für den vorderen Augenabschnitt und einem Pfad für den hinteren Augenabschnitt umgeschaltet wird, US 7,480,059 B2. Mechanische Umschaltungen haben den Nachteil, dass die Umschaltung Zeit benötigt, während der sich das Messobjekt bewegen kann. Diese Bewegung führt zu Ungenauigkeiten in der räumlichen Zuordnung der Messdaten. Während der Umschaltung können keine Messdaten erfasst werden, so dass sich die Messdauer um die Umschaltdauer verlängert. Weiterhin muss mit einer mechanischen Messbereichsumschaltung der Zeitpunkt der Umschaltung mit der Erfassung der interferometrischen Messung synchronisiert werden.

Aus WO 2016/196463 A1 ist ein OCT-System bekannt, bei dem mit einer ersten Lichtquelle ein erster Strahl mit einer Wellenlänge und mit einer zweiten Lichtquelle ein zweiter Strahl mit einer zweiten Wellenlänge abgegeben wird. In einer Variante werden der erste Strahl und der zweite Strahl mit einer Lichtquelle erzeugt.

Der Erfindung liegt die Aufgabe zugrunde, ein OCT-System und ein OCT-Verfahren vorzustellen, so dass bei geringem Aufwand zwei getrennte Messbereiche erfasst werden können. Ausgehend vom genannten Stand der Technik wird die Aufgabe gelöst mit den Merkmalen der unabhängigen Ansprüche. Vorteilhafte Ausführungsformen sind in den Unteransprüchen angegeben.

Bei dem erfindungsgemäßen OCT-System gemäß Anspruch 1 ist in dem OCT-Strahlengang ein wellenlängenabhängiger Strahlenteiler angeordnet, so dass ein erster spektraler Teilstrahl entlang einer längeren Wegstrecke geleitet wird und dass ein zweiter spektraler Teilstrahl entlang einer kürzeren Wegstrecke geleitet wird.

Das OCT-System umfasst eine einheitliche OCT-Lichtquelle, die das OCT-Licht für den ersten spektralen Teilstrahl und den zweiten spektralen Teilstrahl bereitstellt. Das OCT-System umfasst einen einheitlichen Detektor, mit dem das aus dem ersten spektralen Teilstrahl erzeugte Interferenzsignal und das aus dem zweiten spektralen Teilstrahl erzeugte Interferenzsignal aufgenommen werden.

Indem das OCT-Licht mit einem wellenlängenabhängigen Strahlenteiler aufgeteilt wird, wird es möglich, verschiedene Frequenzbereiche des OCT-Lichts ohne mechanische Umschaltung entlang zweier verschiedener Wegstrecken zu leiten. Damit können in einem einheitlichen Messvorgang zwei voneinander getrennte Messbereiche erfasst werden.

Das OCT-System kann einen Trenn-Strahlenteiler aufweisen, mit dem das von der OCT-Lichtquelle ausgesendete OCT-Licht in den Objektstrahlengang und den Referenzstrahlengang aufgespalten wird. Der Trenn-Strahlenteiler ist vorzugsweise nicht wellenlängenabhängig, so dass die Aufspaltung in den Objektstrahlengang und den Referenzstrahlengang das gesamte von der OCT-Lichtquelle abgegebene Spektrum umfasst.

Das OCT-System kann einen Interferenz-Strahlenteiler umfassen, in dem das OCT-Licht aus dem Objektstrahlengang und das OCT-Licht aus dem Referenzstrahlengang zur Interferenz gebracht werden. Der Trenn-Strahlenteiler und der Interferenz-Strahlenteiler können zwei voneinander separate optische Bauteile sein. In einer Ausführungsform sind der Trenn-Strahlenteiler und der Interferenz-Strahlenteiler in einem optischen Bauteil verwirklicht. Die Aufspaltung in den Objektstrahlengang und den Referenzstrahlengang kann in diesem Fall erfolgen, wenn das OCT-Licht in einer Richtung auf das optische Bauteil trifft. Das Interferenzsignal kann erzeugt werden, wenn das OCT-Licht in anderer Richtung, insbesondere in entgegengesetzter Richtung auf das optische Bauteil trifft.

Der Objektstrahlengang kann sich von dem Trenn-Strahlenteiler über das Messobjekt zu dem Interferenz-Strahlenteiler erstrecken. Der Referenzstrahlengang kann sich von dem Trenn-Strahlenteiler über einen Referenzspiegel bis zu dem Interferenz-Strahlenteiler erstrecken. Möglich ist auch, dass der Referenzstrahlengang sich entlang einem Lichtleiter von dem Trenn-Strahlenteiler zu dem Interferenz-Strahlenteiler erstreckt.

Der OCT-Strahlengang kann einen Parallelabschnitt umfassen, in dem der erste spektrale Teilstrahl entlang einer ersten Parallelstrecke geleitet wird und in dem der zweite spektrale Teilstrahl entlang einer zweiten Parallelstrecke geleitet wird. Die erste Parallelstrecke kann länger sein als die zweite Parallelstrecke. Nach dem Austritt aus dem Parallelabschnitt können der erste spektrale Teilstrahl und der zweite spektrale Teilstrahl wieder vereinigt werden, wobei die beiden spektralen Teilstrahlen nach dem Austritt aus dem Parallelabschnitt koaxial oder nicht koaxial sein können. Der Parallelabschnitt kann einen Abschnitt des Objektstrahlengangs bilden oder einen Abschnitt des Referenzstrahlengangs bilden. Der Begriff OCT-Strahlengang wird als Oberbegriff für den Objektstrahlengang und den Referenzstrahlengang verwendet.

Ein erster wellenlängenabhängiger Strahlenteiler kann am Eingang des Parallelabschnitts angeordnet sein. Ein erster spektraler Anteil des OCT-Lichts kann von dem ersten wellenlängenabhängigen Strahlenteiler auf die erste Parallelstrecke geleitet werden, während ein zweiter spektraler Anteil des OCT-Lichts auf die zweite Parallelstrecke geleitet wird.

Ein zweiter wellenlängenabhängiger Strahlenteiler kann am Ausgang des Parallelabschnitts angeordnet sein. Beim Austritt aus dem Parallelabschnitt können die beiden spektralen Teilstrahlen von dem zweiten wellenlängenabhängigen Strahlenteiler wieder zu einem gemeinsamen OCT-Strahlengang vereinigt werden. Die optischen Eigenschaften der beiden wellenlängenabhängigen Strahlenteiler können identisch sein.

Der OCT-Strahlengang kann so gestaltet sein, dass der Parallelabschnitt zweimal durchlaufen wird. Beim zweiten Durchlauf kann die Reihenfolge, in der das OCT-Licht auf die wellenlängenabhängigen Strahlenteiler trifft, umgekehrt sein wie bei dem ersten Durchlauf. Beim zweiten Durchlauf kann die Aufspaltung des OCT-Strahlengangs an dem zweiten wellenlängenabhängigen Strahlenteiler erfolgen und die Vereinigung der beiden Teilstrahlen am ersten wellenlängenabhängigen Strahlenteiler.

Um innerhalb des Parallelabschnitts die Wegstrecke für die beiden spektralen Teilstrahlen verschieden lang zu gestalten, kann innerhalb des Parallelabschnitts ein Teilstrahl-Spiegel angeordnet sein. Als Teilstrahl-Spiegel wird ein Spiegel bezeichnet, der in dem Strahlengang eines der spektralen Teilstrahlen, nicht aber in dem Strahlengang des anderen spektralen Teilstrahls angeordnet ist. Als Spiegel wird allgemein ein optisches Element bezeichnet, mit dem die Richtung eines Strahlengangs umgelenkt wird.

Der Parallelabschnitt kann zwei Teilstrahl-Spiegeln umfassen, mit denen der erste spektrale Teilstrahl zwischen den wellenlängenabhängigen Strahlenteilern um 180° umgelenkt wird. Der zweite spektrale Teilstrahl kann einen geraden Weg zwischen den beiden wellenlängenabhängigen Strahlenteilern nehmen.

Die Weglängendifferenz zwischen der ersten Parallelstrecke und der zweiten Parallelstrecke kann einstellbar sein. Dies kann dadurch verwirklicht werden, dass der Abstand zwischen einem Teilstrahl-Spiegel des Parallelabschnitts und einem wellenlängenabhängigen Strahlenteiler verändert wird. Es kann der Teilstrahl-Spiegel in einer festen Position angeordnet sein und der wellenlängenabhängige Strahlenteiler bewegt werden. Alternativ kann der wellenlängenabhängige Strahlenteiler in einer festen Position angeordnet sein und der Teilstrahl-Spiegel verschiebbar sein. In beiden Fällen kann entweder die längere Wegstrecke des ersten spektralen Teilstrahls oder die kürzere Strecke des zweiten spektralen Teilstrahls in der Länge einstellbar sein. Möglich ist auch, dass die beiden Elemente unabhängig voneinander verschoben werden können. Das Verstellen des Abstands kann manuell oder motorisch angetrieben werden.

In einer Ausführungsform umfasst der Parallelabschnitt einen ersten Teilstrahl-Spiegel und einen zweiten Teilstrahl-Spiegel. Für das Einstellen der Weglängendifferenz können der Abstand zwischen dem ersten Teilstrahl-Spiegel und dem ersten wellenlängenabhängigen Strahlenteiler sowie der Abstand zwischen dem zweiten Teilstrahl-Spiegel und dem zweiten wellenlängenabhängigen Strahlenteiler simultan verändert werden. Simultan einstellbar bedeutet, dass die beiden Abstände jeweils um den gleichen Betrag geändert werden. Die Abstände können gleich sein.

Das Messobjekt, an dem das OCT-System verwendet wird, kann ein Auge sein, insbesondere ein menschliches Auge. Der Verstellbereich der optischen Elemente in dem Parallelabschnitt des OCT-Strahlengangs kann so bemessen sein, dass in einer ersten Konfiguration des Parallelabschnitts der mit dem ersten spektralen Teilstrahl untersuchte erste Objektbereich und der mit dem zweiten spektralen Teilstrahl untersuchte zweite Objektbereich beide in einem vorderen Augenabschnitt angeordnet sind. Der erste Objektbereich kann die Cornea des Auges umfassen. Der zweite Objektbereich kann die Augenlinse des Auges umfassen. In einer zweiten Konfiguration des Parallelabschnitts kann der erste Objektbereich in einem vorderen Augenabschnitt angeordnet sein und der zweite Objektbereich in einem hinteren Augenabschnitt angeordnet sein.

Das OCT-System kann so eingerichtet sein, dass in einem ersten Zustand ein wellenlängenabhängiger Strahlenteiler in dem OCT-Strahlengang angeordnet ist und dass in einem zweiten Zustand der wellenlängenabhängige Strahlenteiler nicht in dem OCT-Strahlengang angeordnet ist. Dies gilt vorzugsweise für alle wellenlängenabhängigen Strahlenteiler, die in dem ersten Zustand in dem OCT-Strahlengang angeordnet sind. Ohne wellenlängenabhängigen Strahlenteiler im OCT-Strahlengang entfällt der Parallelabschnitt und das gesamte Spektrum des OCT-Lichts legt dieselbe Wegstrecke zurück. Mit dem OCT-System kann dann ein einzelner Objektbereich untersucht werden, wobei die axiale Auflösung erhöht ist im Vergleich mit der parallelen Untersuchung von zwei Objektbereichen.

Das OCT-System kann ein die wellenlängenabhängigen Strahlenteiler umfassendes Schaltmodul aufweisen, mit dem zwischen dem ersten Zustand und dem zweiten Zustand des OCT-Systems umgeschaltet wird. Die Schaltbewegung beim Wechsel zwischen den Zuständen kann eine bezogen auf die optische Achse laterale Bewegung sein. Dabei kann das Schaltmodul eine Schwenkbewegung und/oder eine translatorische Bewegung durchführen. Die Bewegung kann manuell oder motorisch angetrieben sein. Das Schaltmodul kann zusätzlich entlang der optischen Achse verschiebbar sein, um die Weglängendifferenz zwischen den beiden beiden spektralen Teilstrahlen einzustellen.

In einer Ausführungsform ist Schaltbewegung beim Wechsel zwischen dem ersten Zustand und dem zweiten Zustand eine zur optischen Achse parallele Bewegung, wobei das Schaltmodul so ausgelegt ist, dass die zur optischen Achse parallele Bewegung in eine laterale Bewegung der wellenlängenabhängigen Strahlenteiler umgesetzt wird. Dies hat den Vorteil, dass ein einziger Aktor ausreichen kann, um sowohl die Weglängendifferenz einzustellen als auch zwischen dem ersten Zustand und dem zweiten Zustand zu wechseln. Beispielsweise kann das Schaltmodul, das zum Einstellen der Weglängendifferenz parallel zur optischen Achse verschoben wird, am Ende des Verstellbereichs an einen Anschlag anstoßen, so dass beim weiteren Verschieben des Schaltmoduls eine Bewegung innerhalb des Schaltmoduls ausgelöst wird, mit der die wellenlängenabhängigen Strahlenteiler aus dem OCT-Strahlengang entfernt werden. Die Bewegung kann eine Schwenkbewegung einer Strahlenteiler-Einheit sein, die die beiden wellenlängenabhängigen Strahlenteiler umfasst. Wünschenswert ist, dass die Strahlenteiler-Einheit eine exakt definierte Position hat, wenn die wellenlängenabhängigen Strahlenteiler in dem OCT-Strahlengang angeordnet sind. Beispielsweise kann die Strahlenteiler-Einheit durch eine Feder und/oder durch einen Magneten in ihrer Position innerhalb des Schaltmoduls gehalten werden.

Der OCT-Strahlengang kann beim Auftreffen auf den ersten wellenlängenabhängigen Strahlenteiler in einem kollimierten Zustand sein. Zwischen dem Trenn-Strahlenteiler und dem ersten wellenlängenabhängigen Strahlenteiler kann eine Kollimationsoptik angeordnet sein, die den OCT-Strahlengang in diesen Zustand bringt. Der Parallelabschnitt des OCT-Strahlengangs kann so gestaltet sein, dass der Strahlengang umgelenkt, aber nicht in seiner Form verändert wird. Beim Verlassen des Parallelabschnitts können die beiden spektralen Teilstrahlen weiterhin in einem kollimierten Zustand sein. Wenn der Parallelabschnitt im Objektstrahlengang angeordnet ist, kann zwischen dem Parallelabschnitt und dem Messobjekt eine Objektivlinse angeordnet sein, mit der die beiden spektralen Teilstrahlen im Objektbereich fokussiert werden. Der Fokus der beiden spektralen Teilstrahlen kann in derselben Objektebene liegen.

Wenn die beiden spektralen Teilstrahlen dazu verwendet werden, Bildinformationen aus zwei Objektbereichen zu gewinnen, die in Axialrichtung voneinander beabstandet sind, kann es von Vorteil sein, wenn die axiale Fokuslage des ersten spektralen Teilstrahls von der axialen Fokuslage des zweiten spektralen Teilstrahls abweicht. Axiale Fokuslage bezeichnet die Fokuslage entlang der optischen Achse der Objektivlinse. Das OCT-System kann eine Teilstrahl-Linse umfassen, die im Strahlengang eines der beiden spektralen Teilstrahlen, nicht aber in dem Strahlengang des anderen der beiden spektralen Teilstrahls angeordnet ist. Der Begriff Linse steht allgemein für ein lichtbrechendes optisches Bauteil. Die Teilstrahl-Linse kann einstellbar sein, so dass die Fokuslage des betreffenden spektralen Teilstrahls in Axialrichtung verschoben werden kann. Beispielsweise kann die Teilstrahl-Linse entlang der optischen Achse verschiebbar sein. Möglich ist auch ein in fester Position angeordnete Linse mit variabler Brechkraft, beispielsweise in Form einer Flüssiglinse.

Die Teilstrahl-Linse kann so angeordnet sein, dass sie zusammen mit der Objektivlinse einen Strahlengang formt, der keinen im Objektbereich liegenden Fokus hat. Beispielweise kann der Strahlengang kollimiert sein. Möglich sind auch eine leicht divergente oder eine leicht konvergente Form, wobei in letzterem Fall der Fokus mindestens 20 cm hinter dem Objektbereich liegt. Insbesondere kann die Teilstrahl-Linse so angeordnet sein, dass sie den spektralen Teilstrahl in einer geräteseitigen Fokusebene der Objektivlinse fokussiert. Möglich ist auch eine umgekehrte Gestaltung, bei der die Objektivlinse alleine den Strahlengang nicht im Objektbereich fokussiert und bei der die Teilstrahl-Linse zusammen mit der Objektivlinse einen im Objektbereich fokussierten Teilstrahl bildet.

Ein solches OCT-System ist insbesondere für Messungen am Auge geeignet. Mit dem im Objektbereich fokussierten Teilstrahl können Bildinformationen des vorderen Augenabschnitts gewonnen werden. Der nicht-fokussierte bzw. kollimierte Strahlengang kann in das Auge eintreten und durch die Cornea und die Augenlinse im hinteren Augenabschnitt fokussiert werden. Es können also sowohl der vordere Augenabschnitt als auch der hintere Augenabschnitt mit einem fokussierten OCT-Strahl untersucht werden.

Das OCT-System kann eine Scaneinrichtung umfassen, um den Objektstrahlengang in seitlicher Richtung abzulenken. Durch Ablenken in seitlicher Richtung können Schnittbilder des Messobjekts erzeugt werden. Wenn die Scaneinrichtung dazu ausgelegt ist, den Objektstrahlengang in zwei seitliche Richtungen abzulenken (zum Beispiel X-Richtung, Y-Richtung), kann aus einer Mehrzahl von Schnittbildern ein dreidimensionales Volumenbild zusammengesetzt werden. Die Scaneinrichtung kann im Gesamtstrahl des Objektstrahlengangs angeordnet sein, also außerhalb des Parallelabschnitts.

Die Scaneinrichtung kann beispielsweise zwei Scanspiegel umfassen, die um zueinander orthogonale Achsen schwenkbar sind. Eine solche Anordnung von Scanspiegeln ist ein gängiges Beispiel einer Scaneinrichtung, mit der ein Messobjekt abgetastet werden kann. Die Scaneinrichtung kann alternativ auch einen einzelnen Spiegel umfassen, der entlang zweier nicht-paralleler Achsen verkippbar ist. Die nicht-parallelen Achsen können orthogonal zueinander oder nicht-orthogonal zueinander sein. Die Scaneinrichtung kann zwischen einer Kollimationsoptik und einer Objektivlinse des Objektstrahlengangs angeordnet sein. Die Optik des Objektstrahlengangs kann telezentrisch ausgelegt sein, so dass die Scaneinrichtung in einem Brennpunkt der Objektivlinse angeordnet ist und der Strahlengang zwischen dem Objektiv und dem Messobjekt beim Scannen parallel verschoben wird. Das parallele Verschieben kann sich auf den ersten spektralen Teilstrahl und den zweiten spektralen Teilstrahl beziehen.

Bei einer Verwendung des OCT-Systems am menschlichen Auge wird der erste spektrale Teilstrahl, der in dem Parallelabschnitt den längeren Weg zurücklegt, normalerweise zum Abtasten des vorderen Augenabschnitts verwendet. Der zweite spektrale Teilstrahl, der in dem Parallelabschnitt den kürzeren Weg zurücklegt, wird zum Abtasten des hinteren Augenabschnitts verwendet. Wird der zweite spektrale Teilstrahl gemeinsam mit dem ersten spektralen Teilstrahl parallel versetzt, so wird der zweite spektrale Teilstrahl trotz der Parallelverschiebung durch die Cornea und die Augenlinse immer auf denselben Bereich der Retina geleitet.

Um eine Bildgebung auch des hinteren Augenabschnitts zu ermöglichen, kann das OCT-System so eingerichtet sein, dass der zweite spektrale Teilstrahl unter einem anderen Winkel auf das Messobjekt auftrifft als der erste spektrale Teilstrahl. Insbesondere kann der erste spektrale Teilstrahl parallel zur optischen Achse des Objektivs auf das Messobjekt geleitet werden. Dies kann für jede Stellung der Scaneinrichtung gelten. Der zweite spektrale Teilstrahl kann unter einem anderen Winkel auf das Messobjekt treffen. Der Winkel kann sich in Abhängigkeit von der Stellung der Scaneinrichtung ändern. Es kann eine Stellung der Scaneinrichtung geben, in der auch der zweite spektrale Teilstrahl parallel zur optischen Achse des Objektivs auf das Messobjekt trifft.

Das OCT-System kann zu diesem Zweck eine außerhalb des Parallelabschnitts angeordnete Gesamtstrahl-Linse umfassen, die die beiden spektralen Teilstrahlen innerhalb des Parallelabschnitts fokussiert. Es ergeben sich ein erster Fokuspunkt in der Parallelstrecke des ersten spektralen Teilstrahls sowie ein zweiter Fokuspunkt in der Parallelstrecke des zweiten spektralen Teilstrahls. Die Gesamtstrahl-Linse kann zwischen dem Trenn-Strahlenteiler und dem Parallelabschnitt angeordnet sein. Die Gesamtstrahl-Linse kann so angeordnet sein, dass der OCT-Strahlengang in kollimiertem Zustand auf die Gesamtstrahl-Linse trifft.

In einer Ausführungsform ist die Gesamtstrahl-Linse zwischen der Scaneinrichtung und dem Parallelabschnitt angeordnet. Außerdem kann die Gesamtstrahl-Linse zwischen dem Trenn-Strahlenteiler und dem Parallelabschnitt angeordnet sein. Durch Betätigen der Scaneinrichtung werden der erste Fokuspunkt und der zweite Fokuspunkt jeweils lateral verschoben. In der Parallelstrecke des ersten spektralen Teilstrahls kann eine Teilstrahl-Linse angeordnet sein, die den ersten spektralen Teilstrahl wieder in einen kollimierten Zustand versetzt. Der erste spektrale Teilstrahl kann in kollimiertem Zustand auf die Objektivlinse geleitet werden, so dass der erste spektrale Teilstrahl durch die Objektivlinse im Objektbereich fokussiert wird.

Der zweite spektrale Teilstrahl kann in divergentem Zustand auf die Objektivlinse treffen. Wenn der Abstand zwischen dem zweiten Fokuspunkt und der Objektivlinse der Brennweite der Objektivlinse entspricht, wird der zweite spektrale Teilstrahl durch die Objektivlinse in einen kollimierten Zustand versetzt. Der laterale Versatz des zweiten Fokuspunkts wird durch die Objektivlinse in eine Richtungsänderung umgesetzt. Indem der zweite Fokuspunkt mit der Scaneinrichtung lateral verschoben wird, ändert sich also die Richtung, unter der der zweite spektrale Teilstrahl auf das Messobjekt trifft. Ist das Messobjekt ein Auge, werden die unterschiedlichen Richtungen in unterschiedliche Positionen im hinteren Augenabschnitt übersetzt.

Der wellenlängenabhängige Strahlenteiler kann eine Grenzwellenlänge aufweisen, so dass Wellenlängen oberhalb der Grenzwellenlänge in den einen spektralen Teilstrahl und Wellenlängen unterhalb der Grenzwellenlänge in den anderen spektralen Teilstrahl geleitet werden. Die Grenzwellenlänge kann etwa mittig innerhalb des Frequenzbands des OCT-Lichts liegen. Damit ergibt sich eine etwa gleich gute axiale Auflösung der mit den beiden spektralen Teilstrahlen untersuchten Objektbereiche. Möglich ist auch eine außermittig innerhalb des Frequenzbands angeordnete Grenzwellenlänge, so dass die Auflösung in dem einen Objektbereich auf Kosten der Auflösung in dem anderen Objektbereich erhöht wird. Möglich ist auch die Verwendung eines Strahlenteilers mit einstellbarer Grenzwellenlänge, so dass das Auflösungsvermögen zwischen den beiden Objektbereichen variiert werden kann.

Der OCT-Strahlengang kann als Freistrahl auf den wellenlängenabhängigen Strahlenteiler treffen. Der wellenlängenabhängigen Strahlenteiler kann als dichroitischer Teilerspiegel ausgebildet sein, der einen Teil der Frequenzen des OCT-Lichts reflektiert und einen anderen Teil der Frequenzen des OCT-Lichts transmittiert. Solche Strahlenteiler sind Glassubstrate mit einer dielektrischen Beschichtung, die OCT-Licht abhängig von der Frequenz entweder reflektieren oder transmittieren. Dichroitische Teilerspiegel sind üblicherweise für einen Einfallswinkel von 45° ausgelegt. Besonders geeignet sind dichroitische Teilerspiegel, die innerhalb des Spektrums des OCT-Lichts genau ein Transmissionsband und genau ein Reflektionsband aufweisen. Das Transmissionsband ist das Wellenlängenintervall, in dem der Strahlenteiler überwiegend transmittiert; entsprechend ist das Reflektionsband das Wellenlängenintervall, in dem der Strahlenteiler überwiegend reflektiert. Die Grenzwellenlänge des dichroitischen Strahlenteilers entspricht jener Wellenlänge zwischen Transmissions- und Reflektionsband, für die etwa die gleiche Lichtleistung transmittiert wie reflektiert wird. Das Transmissionsband kann Wellenlängen umfassen, die länger sind als die Grenzwellenlänge, das Reflektionsband umfasst dann Wellenlängen, die kürzer sind als die Grenzwellenlänge ("long-pass"). Eine umgekehrte Konfiguration ("shortpass") ist auch möglich. Besonders gut eignen sich dichroitische Strahlenteiler, die eine hohe Kantensteilheit haben, die also in einem sehr kleinen Wellenlängenintervall von starker Transmission zu starker Reflektion übergehen. Der Strahlenteiler kann beispielsweise innerhalb von höchstens 2% der Kantenwellenlänge von 20% Transmission auf 80% Transmission übergehen. In einer vorteilhaften Ausführungsform geht der Strahlenteiler innerhalb von höchstens 0,7% der Kantenwellenlänge von 10% Transmission auf 90% Transmission über.

Von Vorteil ist weiterhin, wenn der dichroitische Teilerspiegel im Transmissionsband eine möglichst niedrige Reflektion und im Reflektionsband eine möglichst niedrige Transmission hat. Bei zu geringem spektralen Trennkontrast der Strahlengänge werden die Signale der beiden OCT-Messbereiche nur ungenügend getrennt. Gute dichroitische Teilerspiegel erreichen im Reflektionsband eine Transmission < 1% sowie im Transmissionsband eine Reflektion < 5%.

Alternativ zur Freistrahlanordnung können der OCT-Strahlengang und/oder die spektralen Teilstrahlen auch innerhalb von Lichtleitern geführt sein. Der Parallelabschnitt kann zwei zueinander parallele Lichtleiter von unterschiedlicher Länge umfassen, zwischen denen das OCT-Licht durch wellenlängenselektive Umschalter aufgeteilt wird. Ein wellenlängenselektiver Umschalter ist eine alternative Ausführungsform eines wellenlängenabhängigen Strahlenteilers. Ein solches faserbasiertes OCT-System kann einen Umgehungs-Lichtleiter umfassen, der den Parallelabschnitt umgeht. Mit faseroptischen Schaltelementen kann der OCT-Strahlengang zwischen dem Parallelabschnitt und dem Umgehung-Lichtleiter umgeschaltet werden. Tritt der OCT-Strahlengang durch den Parallelabschnitt hindurch, können zwei Objektbereiche mit verminderter Auflösung untersucht werden. Tritt der OCT-Strahlengang durch den Umgehungs-Lichtleiter hindurch, so kann ein Objektbereich mit erhöhter Auflösung untersucht werden.

Die Lichtquelle des OCT-Systems kann eine Swept-Source-Lichtquelle sein, bei der schmalbandiges OCT-Licht innerhalb einer Durchstimmzeit über einen spektralen Durchstimmbereich durchgestimmt wird (Swept-Source-OCT, SS-OCT). Die Swept-Source-Lichtquelle erzeugt mit einem einheitlichen Durchstimmvorgang den ersten spektralen Teilstrahl und den zweiten spektralen Teilstrahl. Das mit dem ersten spektralen Teilstrahl erzeugte Interferenzsignal ist dann zeitlich getrennt von dem mit dem zweiten spektralen Teilstrahl erzeugten Interferenzsignal. Die beiden Interferenzsignale, die mit Photodioden zeitaufgelöst aufgenommen werden, können getrennt voneinander spektral aufgelöst werden. Der Photostrom der Photodioden kann in eine Spannung gewandelt und digitalisiert werden. Anschließend an die spektrale Auflösung kann für beide Interferenzsignale getrennt die Transformation in ein räumliches Signal erfolgen.

In Kombination mit der lateralen Ablenkung des Objektstrahlengangs durch die Scaneinrichtung können für jeden der spektral getrennten gemessenen Objektbereiche Schnittbilder des Messobjekts erstellt werden.

Die Datenerfassung eines SS-OCT Interferometers kann so konfiguriert werden, dass das Interferenzsignal in äquidistanten Wellenlängenintervallen δk erfasst wird (siehe beispielsweise US 2008/0175465). Für eine durchstimmbare Lichtquelle mit einem Durchstimmbereich Δk lässt sich jedem erfassten Messwert innerhalb des Durchstimmvorgangs eine Wellenzahl kᵢ = k₀ + i·δk zuweisen, wobei i Werte zwischen 0 und n = Δk / δk - 1 annehmen kann. Umgekehrt lässt sich das Interferenzsignal als Signalvektor mit einem Positionsindex i beschreiben. Um nun das Interferenzsignal in die beiden spektralen Kanäle (entsprechend den Teilstrahlen) zu trennen, bestimmt man die Wellenzahl der Grenzwellenlänge des dichroitischen Spiegels k_{d} bzw. die Position im Signalvektor, j_{d} = (k_{d} - k₀) / δk. Das spektral aufgelöste Signal für Teilstrahl 1 ist der Signalvektor mit i = 0 ... j_{d} - 1; für Teilstrahl 2 gilt entsprechend i = j_{d} ... n. Die beiden spektral aufgelösten Signale können nun unabhängig voneinander mit bekannten Methoden weiterverarbeitet werden. Die Bearbeitungsschritte umfassen üblicherweise mindestens die Multiplikation mit einer Fensterfunktion, die Multiplikation mit einem komplexwertigen Vektor sowie eine anschließende Fouriertransformation. Je nach Eigenschaften des dichroitischen Strahlteilers kann es auch zweckmäßig sein, nur einen Teil der spektral aufgelösten Signale zu verarbeiten. Insbesondere kann man Interferenzsignale im Übergangsbereich des dichroitischen Strahlteilers verwerfen, um eine ungenügende Kanaltrennung zu vermeiden.

Alternativ kann die Lichtquelle des OCT-Systems eine breitbandige Lichtquelle sein. Als Detektor wird dann ein Spektrometer verwendet (Spectral-Domain-OCT, SD-OCT). Um das mit dem ersten spektralen Teilstrahl erzeugte Interferenzsignal von dem mit dem zweiten spektralen Teilstrahl erzeugten Interferenzsignal zu trennen, wird das vom Spektrometer erfasste Interferenzsignal abzüglich des Referenzarmspektrums zunächst nach bekannten Methoden in eine in Wellenzahl linearisierte Darstellung transformiert. Für die anschließende Auswertung kann entsprechend wie bei der Swept-Source-OCT vorgegangen werden. Als Oberbegriff für SS-OCT und SD-OCT wird der Begriff Fourier-Domain-OCT verwendet.

Die Erfindung betrifft außerdem ein OCT-Verfahren gemäß Anspruch 14, bei dem OCT-Licht ausgesendet wird und in einen Objektstrahlengang und einen Referenzstrahlengang aufgespalten wird. Mit einem Detektor wird ein aus dem Objektstrahlengang und dem Referenzstrahlengang erzeugtes Interferenzsignal aufgenommen. In dem OCT-Strahlengang ist ein wellenlängenabhängiger Strahlenteiler angeordnet, so dass ein erster spektraler Teilstrahl entlang einer längeren Wegstrecke geleitet wird und dass ein zweiter spektraler Teilstrahl entlang einer kürzere Wegstrecke geleitet wird.

Das Verfahren kann mit weiteren Merkmalen fortgebildet werden, die im Zusammenhang des erfindungsgemäßen Systems beschrieben sind. Das System kann mit weiteren Merkmalen fortgebildet werden, die im Zusammenhang des erfindungsgemäßen Verfahrens beschrieben sind.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine erste Ausführungsform eines erfindungsgemäßen OCT-Systems;
- Fig. 2:: das OCT-System aus Fig. 1 in einem anderen Zustand;
- Fig. 3:: das OCT-System aus Fig. 1 in noch einem anderen Zustand;
- Fig. 4:: eine alternative Ausführungsform eines erfindungsgemäßen OCT-Systems;
- Fig. 5:: ein Detail eines erfindungsgemäßen OCT-Systems;
- Fig. 6:: die Ansicht gemäß Fig. 5 bei einer alternativen Ausführungsform;
- Fig. 7-9:: Details erfindungsgemäßer OCT-Systeme bei alternativen Ausführungsformen der Erfindung;
- Fig. 10:: ein Beispiel eines Messobjekts eines erfindungsgemäßen OCT-Systems.

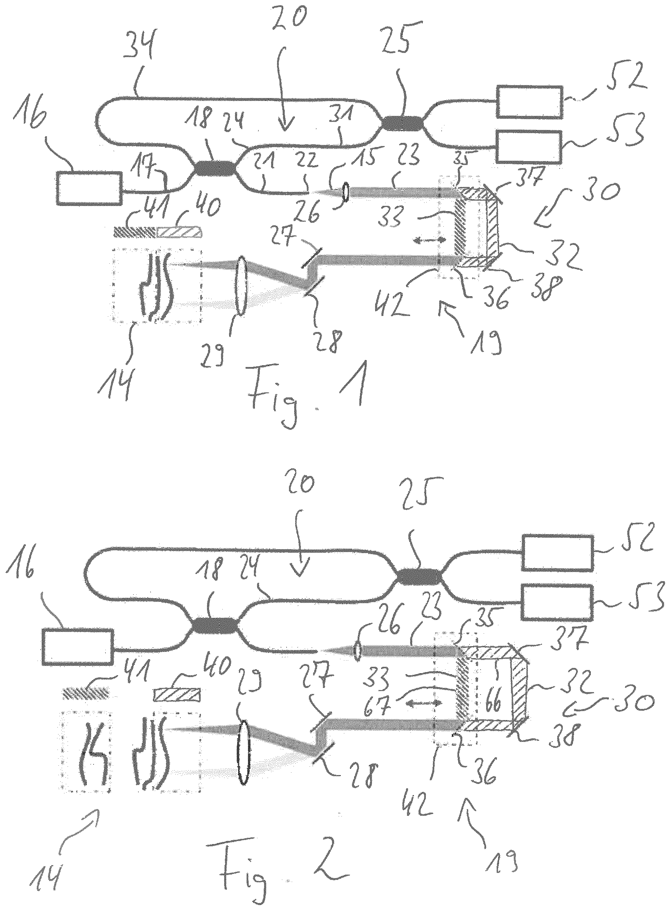

Ein in Fig. 1 gezeigtes OCT-System dient zur Untersuchung eines Messobjekts 14, beispielsweise in Form eines menschlichen Auges. Indem OCT-Licht 15 auf das Messobjekt 14 gerichtet wird, wird eine Bildinformation gewonnen, die sich entlang der Achse des OCT-Strahls in die Tiefe des Messobjekts 14 erstreckt. Indem der OCT-Strahl in einer dazu senkrechten Richtung über das Messobjekt 14 gescannt wird, kann aus einer Vielzahl von einzelnen Messaufnahmen ein dreidimensionales Bild des Messobjekts 14 gewonnen werden.

Das OCT-System umfasst eine OCT-Lichtquelle 16, die als Swept-Source-Lichtquelle ausgebildet ist. Die Swept-Source-Lichtquelle 16 erzeugt schmalbandiges Licht, das spektral durchstimmbar ist. Zu jedem Moment wird also schmalbandiges Licht ausgesendet, dessen Frequenz sich mit der Zeit ändert, so dass die Swept-Source-Lichtquelle während einer Durchstimmzeit über einen Frequenzbereich durchgestimmt wird.

Das von der OCT-Lichtquelle 16 abgegebene OCT-Licht 15 wird in einen ersten Lichtleiter 17 eingespeist, der als Monomoden-Lichtleiter ausgebildet ist. Der erste Lichtleiter 17 erstreckt sich zu einem Trenn-Strahlenteiler 18 in Form eines Faserkopplers, in dem das OCT-Licht 15 aus dem ersten Lichtleiter 17 in einen Objektstrahlengang 23 und einen Referenz-Strahlengang 24 aufgespalten wird. Der Objektstrahlengang 23 erstreckt sich von dem Trenn-Strahlenteiler 18 entlang einem Objektarm 19 bis zu dem Messobjekt 14. Der Referenzstrahlengang 24 erstreckt sich von dem Trenn-Strahlenteiler 18 entlang einem Referenzarm 20 bis zu einem Interferenz-Strahlenteiler 25.

Der Objektarm 19 umfasst einen zweiten Lichtleiter 21, der sich von dem Faserkoppler 18 bis zu einem Austrittsende 22 erstreckt. An dem Austrittsende 22 tritt der Objektstrahlengang 23 in divergentem Zustand aus dem zweiten Lichtleiter 21 aus und wird mit einer Kollimationslinse 26 in einen kollimierten Zustand gebracht.

Eine Scaneinrichtung umfasst zwei Scanspiegel 27, 28, die um zwei zueinander orthogonale Achsen schwenkbar sind. Der Objektstrahlengang 23 wird über die Scaneinrichtung 27, 28 zu einer Objektivlinse 29 geleitet. Der Objektstrahlengang 23 tritt durch die Objektivlinse 29 hindurch und wird im Bereich des Messobjekts 14 fokussiert. Der Abstand zwischen der Objektivlinse 29 und dem zweiten Scanspiegel 28 entspricht der Brennweite der Objektivlinse 29, so dass das Messobjekt 40 unabhängig vom Abstand zur Objektivlinse 29 durch lateral versetzte Messstrahlen abgetastet wird. Eine solche Anordnung aus Objektivlinse 29 und Scaneinrichtung 27, 28 wird als telezentrisch bezeichnet.

Durch Schwenken der Scanspiegel 27, 28 ändert sich die Richtung, unter der der Objektstrahlengang 23 auf die Objektivlinse 29 auftrifft. Da der zweite Scanspiegel 28 im Brennpunkt der Objektivlinse 29 angeordnet ist, erstreckt der Strahlengang 23 sich zwischen der Objektivlinse 29 und dem Messobjekt 14 unabhängig von der Stellung des Scaneinrichtung 27, 28 parallel zur optischen Achse der Objektivlinse 29.

Zwischen der Kollimationslinse 26 und der Scaneinrichtung 27, 28 ist ein Parallelabschnitt 30 des Objektstrahlengangs 23 angeordnet, in dem ein erster spektraler Teilstrahl 32 und ein zweiter spektraler Teilstrahl 33 des Objektstrahlengangs entlang verschiedenen langer Wege geführt werden. Vor dem Eintritt in den Parallelabschnitt 30 und nach dem Austritt aus dem Parallelabschnitt 30 sind die Wege der beiden spektralen Teilstrahlen 32, 33 identisch.

Von dem Messobjekt 14 zurückgeworfenes OCT-Licht bewegt sich mit entgegengesetzter Ausbreitungsrichtung entlang dem Objektarm 19 zurück zu dem Trenn-Strahlenteiler 18 und durch den Trenn-Strahlenteiler 18 hindurch entlang einem dritten Lichtleiter 34 bis zu dem Interferenz-Strahlenteiler 25.

Der Referenzarm 20 umfasst einen vierten Lichtleiter 31, der sich von dem Trenn-Strahlenteiler 18 bis zu dem Interferenz-Strahlenteiler 25 erstreckt. Der in Fig. 1 verkürzt dargestellte vierte Lichtleiter 31 ist so bemessen, dass die optische Weglänge zwischen dem Trenn-Strahlenteiler 18 und dem Interferenz-Strahlenteiler 25 im Objektarm 19 und im Referenzarm 20 gleich lang ist. In dem Interferenz-Strahlenteiler 25 werden der Objektstrahlengang 23 und der Referenzstrahlengang 24 wieder zusammengeführt, so dass ein Interferenzsignal entsteht. Das Interferenzsignal ist desto stärker, je mehr OCT-Licht von einer bestimmten Struktur innerhalb des Messobjekts 14 zurückgeworfen wird. Durch Auswerten des Interferenzsignals können also Streuzentren innerhalb des Messobjekts 14 identifiziert werden.

Ist ein Streuzentrum gerade an dem Referenzpunkt des Objektstrahlengangs angeordnet, so sind der optische Weg des Objektstrahlengangs 23 und des Referenzstrahlengangs 24 exakt gleich lang, so dass sich ein stehendes Interferenzsignal ergibt. Ist das Streuzentrum von dem Referenzpunkt entfernt, so oszilliert das Interferenzsignal (in spektraler Darstellung), wobei die Frequenz je größer wird, desto größer der Abstand zum Referenzpunkt ist.

Mit zwei Detektor-Elementen 52, 53, die Bestandteile eines einheitlichen Detektors im Sinne der Erfindung sind, werden die um 180° phasenverschobenen Interferenzsignale aus dem Interferenz-Strahlenteiler 25 aufgenommen. Durch Differenzbildung zwischen den beiden Detektor-Elementen 52, 53 kann der stationäre Anteil des Signals eliminiert werden, so dass sich ein Nutzsignal mit hoher Auflösung ergibt. Die Differenz der Photoströme der Detektor-Elemente 52, 53 wird in eine Spannung gewandelt und digitalisiert. Durch laterale Ablenkung des OCT-Strahls mit der Scaneinrichtung 27, 28 können Schnittbilder des Messobjekts 14 erstellt werden.

In dem Parallelabschnitt 30 des Objektarms 19 trifft der Objektstrahlengang 23 unter einem Winkel von 45° auf einen wellenlängenabhängigen Teilerspiegel 35 in Form eines dichroitischen Teilerspiegels. In dem dichroitischen Teilerspiegel 35 erfolgt eine spektrale Aufspaltung des OCT-Lichts 15. Lichtanteile, deren Frequenz größer ist als eine Grenzwellenlänge des dichroitischen Teilerspiegels 35 werden reflektiert. Lichtanteile, deren Frequenz kleiner ist als eine Grenzwellenlänge des dichroitischen Teilerspiegels 35 werden transmittiert. Der transmittierte Anteil des OCT-Lichts 15 bildet den ersten spektralen Teilstrahl 32. Der reflektierte Anteil des OCT-Lichts 15 bildet den zweiten spektralen Teilstrahl 33.

Das von der OCT-Lichtquelle 16 abgegebene OCT-Licht 15 kann sich beispielsweise über ein Wellenlängenspektrum von A=1000 nm bis A=1100 nm erstrecken. Der erste dichroitische Teilerspiegel 35 kann so ausgeführt sein, dass er für OCT-Licht mit einer Wellenlänge von weniger als A=1045 nm eine hohe Reflektivität und für OCT-Licht mit einer Wellenlänge von mehr als A=1055 nm eine hohe Transmission aufweist.

Der zweite spektrale Teilstrahl 33 trifft auf einen zweiten wellenlängenabhängigen Teilerspiegel 36, der ebenfalls als dichroitischer Strahlenteiler ausgebildet ist. Der zweite dichroitische Teilerspiegel 36 hat die gleichen optischen Eigenschaften wie der erste dichroitische Teilerspiegel 35. Der erste spektrale Teilstrahl 33 wird also auch an dem zweiten dichroitischen Teilerspiegel 36 reflektiert und in Richtung der Scaneinrichtung 27, 28 umgelenkt.

Der erste spektrale Teilstrahl 32 wird über zwei Spiegel 37, 38 zu dem zweiten dichroitischen Teilerspiegel 36 geleitet, so dass der erste spektrale Teilstrahl 32 innerhalb des Parallelabschnitt 30 einen längeren Weg zurücklegt als der zweite spektrale Teilstrahl 33. Die Spiegel 37, 38, die nur den ersten spektralen Teilstrahl 32, nicht aber den zweiten spektralen Teilstrahl 33 umlenken, sind Teilstrahl-Spiegel 37, 38 im Sinne der Erfindung. In dem zweiten dichroitischen Teilerspiegel 36 werden die beiden spektralen Teilstrahlen 32, 33 wieder vereinigt. Auf dem Rückweg vom Messobjekt 14 zu dem Interferenz-Strahlenteiler 25 nimmt der zweite spektrale Teilstrahl 33 erneut den direkten Weg zwischen den beiden dichroitischen Teilerspiegeln 35, 36, während der erste spektrale Teilstrahl 32 erneut den längeren Weg über die Teilstrahl-Spiegel 37, 38 nimmt. Die Weglängendifferenz zwischen den beiden spektralen Teilstrahlen 32, 33 verdoppelt sich also mit dem zweimaligen Durchlaufen des Parallelabschnitts 30.

Die Weglängendifferenz zwischen dem ersten spektralen Teilstrahl 32 und dem zweiten spektralen Teilstrahl 33 führt dazu, dass das Interferenzsignal sich auf Strukturen des Messobjekts 14 bezieht, die axial voneinander beabstandet sind. Der erste spektrale Teilstrahl 32, der in dem Parallelabschnitt 30 einen längeren Weg zurücklegt, erzeugt ein Interferenzsignal aus einem ersten Objektbereich 40, der einen geringeren Abstand zu der Objektivlinse 29 hat. Der zweite spektrale Teilstrahl 33, der in dem Parallelabschnitt 30 einen kürzeren Weg zurücklegt, erzeugt ein Interferenzsignal aus einem zweiten Objektbereich 41, der einen größeren Abstand zu der Objektivlinse 29 hat. Da die Swept-Source-Lichtquelle 16 die verschiedenen Frequenzen über die Zeit verteilt abgibt, sind die Interferenzsignale aus den Objektbereichen 40, 41 zeitlich voneinander getrennt. Die Bildinformationen können entsprechend der zeitlichen Trennung getrennt ausgewertet werden. Dazu wird das Interferenzsignal zunächst spektral aufgelöst digitalisiert und anschließend in ein räumliches Signal transformiert. Bei der SD-OCT, bei der alle Frequenzen gleichzeitig auf das Objekt treffen, wird das vom Spektrometer erfasste Interferenzsignal abzüglich des Referenzarmspektrums nach bekannten Verfahren in eine in Wellenzahl linearisierte Darstellung transformiert, bevor die nach den Objektbereichen getrennte Auswertung erfolgt.

Ein Schaltmodul 42, das die beiden dichroitischen Teilerspiegeln 35, 36 umfasst, ist verschiebbar gelagert, so dass der Abstand zwischen den beiden dichroitischen Teilerspiegeln 35, 36 und den beiden Teilstrahl-Spiegeln 37, 38 einstellbar ist. Das Verschieben des Schaltmoduls 42 kann manuell oder motorisch erfolgen. In Fig. 2 ist ein Zustand des OCT-Systems gezeigt, in dem die beiden dichroitischen Teilerspiegel 35, 36 einen größeren Abstand zu den beiden Teilstrahl-Spiegeln 37, 38 haben als in Fig. 1. Die Lage des mit dem ersten spektralen Teilstrahl 32 abgetasteten ersten Objektbereichs 40 bleibt unverändert. Die Lage des mit dem zweiten spektralen Teilstrahl 33 abgetasteten zweiten Objektbereichs ist nach hinten verschoben, so dass der Abstand zwischen den beiden Objektbereichen 40, 41 vergrößert ist. Sowohl in Fig. 1 als auch in Fig. 2 entspricht der Abstand zwischen den beiden Objektbereichen 40, 41 dem Abstand von zwischen dem ersten dichroitischen Teilerspiegel 35 und dem ersten Teilstrahl-Spiegel 37 plus dem Abstand zwischen dem zweiten dichroitischen Teilerspiegel 36 und dem zweiten Teilstrahl-Spiegel 38.

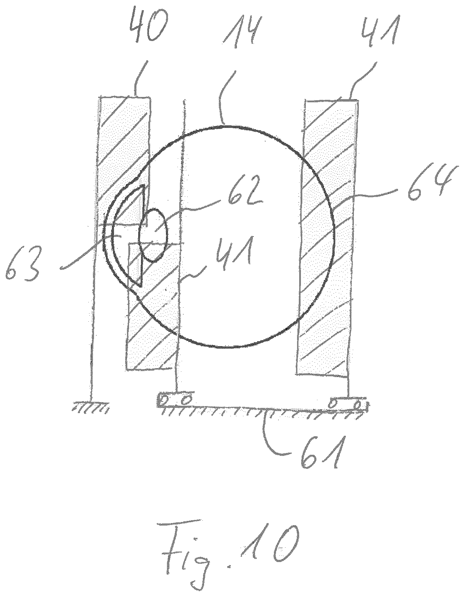

In Fig. 10 ist ein menschliches Auge dargestellt, das als Messobjekt 14 des OCT-Systems dienen kann. Der erste Objektbereich 40 des OCT-Systems wird so positioniert, dass er die Cornea 63 des Auges überdeckt. Für den zweiten Objektbereich 41 sind die beiden Varianten gemäß den Fig. 1 und 2 dargestellt. Sind die beiden Objektbereiche 40, 41 gemäß Fig. 1 unmittelbar miteinander benachbart, so überdeckt der zweite Objektbereich 41 die Augenlinse 62. Sind die beiden Objektbereiche 40, 41 voneinander beabstandet, wie in Fig. 2 gezeigt, so kann mit dem zweiten Objektbereich 41 die Retina 64 des Auges untersucht werden. Der Verschiebemechanismus 61, mit dem das Schaltmodul 42 zwischen den Positionen verschoben werden kann, ist in Fig. 10 schematisch angedeutet. Wird der Verschiebemechanismus 61 über einen Anschlag hinaus betätigt, wird die Teilerspiegel-Einheit 42 mit einer Schwenkbewegung aus dem Objektstrahlengang 23 entfernt.

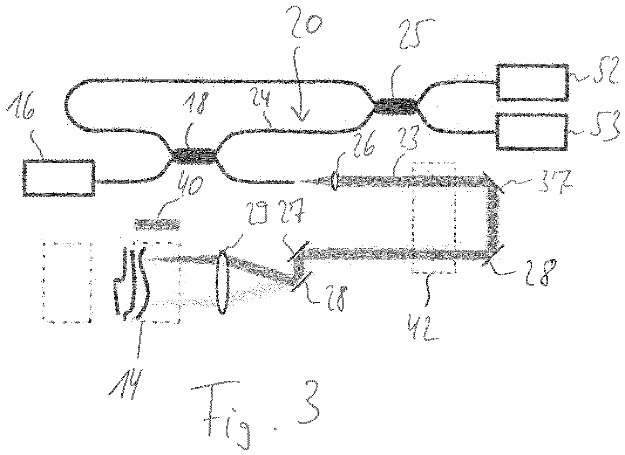

In Fig. 3 ist das OCT-System in einem Zustand dargestellt, in dem die Teilerspiegel-Einheit 42 aus dem Objektstrahlengang 23 entfernt ist. Das OCT-Licht nimmt in seiner Gesamtheit den Weg des ersten spektralen Teilstrahls 32, den ansonsten nur das von den dichroitischen Teilerspiegeln 35, 36 transmittierte Licht nimmt. Ein Interferenzsignal entsteht nur durch das von dem ersten Objektbereich 40 zurückgestreute Licht. Aufgrund der höheren Bandbreite des OCT-Lichts ist die Bildauflösung verbessert verglichen mit einer Bildinformation, die nur aus dem ersten spektralen Teilstrahl 32 abgeleitet wird. Es kann ein Schwenkmechanismus vorgesehen sein, mit dem das Schaltmodul 42 in den Objektstrahlengang dann 20 eingeschwenkt bzw. aus diesem ausgeschwenkt werden kann. Die Schwenkbewegung des Schaltmoduls 42 kann manuell oder motorisch angetrieben sein.

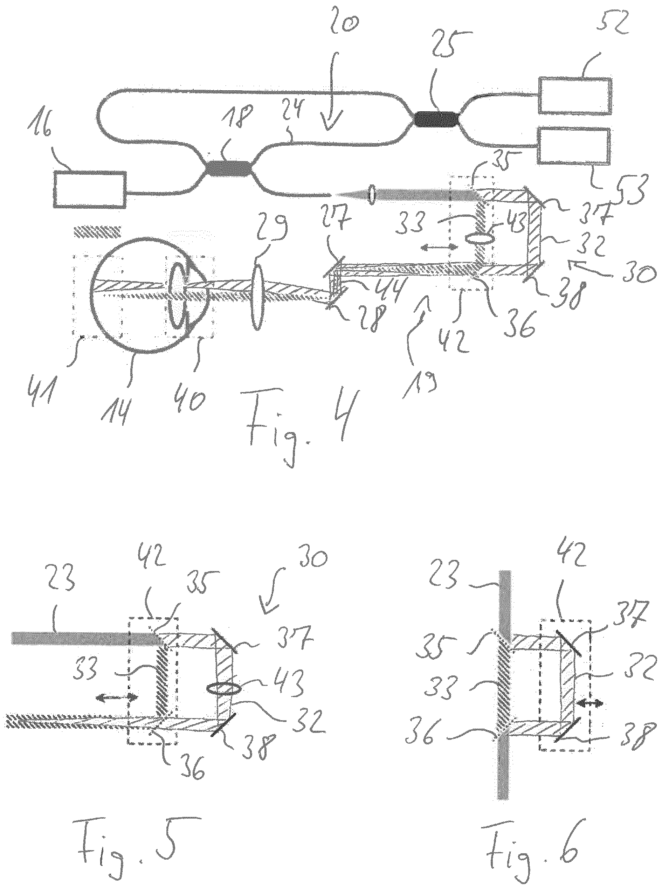

Bei dem in Fig. 4 dargestellten OCT-System entspricht der erste Objektbereich 40 dem vorderen Abschnitt und der zweite Objektbereich 41 dem hinteren Abschnitt eines menschlichen Auges. Das OCT-System ist so vor dem Auge positioniert, dass der erste spektrale Teilstrahl 32 auf den vorderen Augenabschnitt 40 fokussiert ist.

In dem zweiten spektralen Teilstrahl 33 ist zwischen den beiden dichroitischen Teilerspiegeln 35, 36 eine Teilstrahl-Linse 43 angeordnet, die den zweiten spektralen Teilstrahl 33 auf einen Fokuspunkt 44 fokussiert, der in diesem Beispiel zwischen den beiden Scanspiegeln 27, 28 angeordnet ist. Der Abstand zwischen dem Fokuspunkt 44 und der Objektivlinse 29 entspricht der Brennweite der Objektivlinse 29, so dass der zweite spektrale Teilstrahl 32 beim Durchtreten durch die Objektivlinse 29 in einen kollimierten Zustand gebracht wird. Durch die Brechtkraft der Hornhaut und der Augenlinse des Auges 14 wird der zweite spektrale Teilstrahl 33 auf den hinteren Augenabschnitt 41 fokussiert. Mit diesem OCT-System können also sowohl vom vorderen Augenabschnitt 40 als auch vom hinteren Augenabschnitt 41 scharf aufgelöste Messwerte gewonnen werden. Die Teilstrahl-Linse 43 kann als Linse mit variabler Brechkraft gestaltet sein, beispielsweise in Form einer Flüssiglinse. Damit wird es möglich, den Fokus des Strahlengangs je nach Messbereichslage auf den vorderen Augenabschnitt 40 oder den hinteren Augenabschnitt 41 zu legen.

In Fig. 5 ist eine Variante dargestellt, bei der die Teilstrahl-Linse 43 nicht im zweiten spektralen Teilstrahl 33, sondern im ersten spektralen Teilstrahl 32 angeordnet ist. Abhängig von den Eigenschaften und der Anordnung der anderen Linsen im Objektstrahlengang 23 kann diese Position der Teilstrahl-Linse 43 von Vorteil sein.

Bei der weiteren Variante gemäß Fig. 6 sind die Verhältnisse bei den dichroitischen Teilerspiegeln 35, 36 umgekehrt. Der erste spektrale Teilstrahl 32 wird an den dichroitischen Teilerspiegeln 35, 36 reflektiert, während der zweite spektrale Teilstrahl 33 von den dichroitischen Teilerspiegeln 35, 36 transmittiert wird. Durch Verschieben der Einheit 42 aus den Teilstrahl-Spiegeln 37, 38 kann die Weglänge des ersten spektralen Teilstrahls 32 verändert werden. Es bleibt also die Lage des zweiten Objektbereichs 41 unverändert, während die Lage des ersten Objektbereichs 40 verschoben wird. Werden bei dieser Variante die beiden dichroitischen Teilerspiegel 35, 36 aus dem Strahlengang entfernt, so ist eine Messung in dem hinteren Objektbereich 41 mit erhöhter axialer Auflösung möglich.

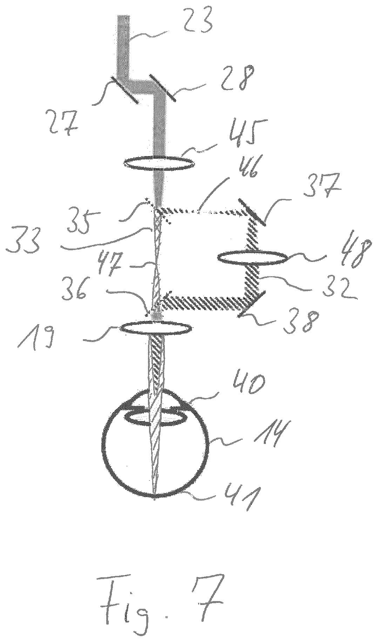

Die Fig. 7 zeigt eine Variante mit einer etwas anderen Gestaltung des Objektarms 19. Die Scaneinrichtung 27, 28 ist vor dem ersten dichroitischen Teilerspiegel 35 angeordnet, zwischen der Scaneinrichtung 27, 28 und dem ersten dichroitischen Teilerspiegel 35 befindet sich eine Linse 45, durch die der gesamte Objektstrahlengang 23 hindurchtritt. Die Linse 45 ist eine Gesamtstrahl-Linse im Sinne der Erfindung.

Die Gesamtstrahl-Linse 45 ist so angeordnet, dass der erste spektrale Teilstrahl 32 in einem ersten Fokuspunkt 46 fokussiert wird, der zwischen dem ersten dichroitischen Teilerspiegel 35 und in dem ersten Teilstrahl-Spiegel 37 angeordnet ist. Eine Teilstrahl-Linse 48 zwischen den Teilstrahl-Spiegeln 37, 38 ist so angeordnet, dass der erste spektrale Teilstrahl 32 beim Durchtritt durch die Teilstrahl-Linse 48 in einen kollimierten Zustand versetzt wird. Mit der Objektivlinse 19 wird der erste Teilstrahl 32 auf den vorderen Augenabschnitt 40 fokussiert.

Der zweite spektrale Teilstrahl 33 wird in einem zweiten Fokuspunkt 47 fokussiert, der zwischen dem ersten dichroitischen Teilerspiegel 35 und dem zweiten dichroitischen Teilerspiegel 36 angeordnet ist. Der Abstand zwischen dem ersten Fokuspunkt 46 und der Objektivlinse 49 entspricht der Brennweite der Objektivlinse 19, so dass der zweite spektrale Teilstrahl 33 beim Durchtritt durch die Objektivlinse 19 in einen kollimierten Zustand versetzt wird. Mit dem Durchtritt durch die Cornea und die Augenlinse wird der zweite spektrale Teilstrahl auf den hinteren Augenabschnitt 41 fokussiert.

Um die Objektbereiche 40, 41 zum Zwecke der Bildgebung in laterale Richtung abzutasten, wird der Objektstrahlengang 23 mit den Scanspiegeln 27, 28 um einen Winkel von der optischen Achse des Systems abgelenkt. Die Gesamtstrahl-Linse 45 fokussiert den Strahl und wandelt dabei den Winkel des Strahls in einen lateralen Versatz der Fokuspunkte 46, 46 in ihrer jeweiligen Fokusebene um. Bei dem zweiten spektralen Teilstrahl 33 wird die laterale Position des zweiten Fokuspunkts 47 durch die Objektivlinse 19 in einen Strahlwinkel umgewandelt, unter dem der zweite spektrale Teilstrahl 33 auf die Pupille des Auges gelenkt wird. Der beim Auftreffen kollimierte Teilstrahl 33 wird durch die Cornea und die Augenlinse auf die Netzhaut fokussiert. Durch Winkeländerung der Scanspiegel 27, 28 tastet der zweite spektrale Teilstrahl 33 die Netzhaut des Auges ab. Der zweite spektrale Teilstrahl 33 ermöglicht also eine Bildgebung im hinteren Augenabschnitt 41.

Analog wird bei dem ersten spektralen Teilstrahl 32 der erste Fokuspunkt 46 in seiner Fokusebene lateral gescannt. Die Teilstrahl-Linse 48 kollimiert den ersten spektralen Teilstrahl 32, der dann von der Objektivlinse 19 auf den vorderen Augenabschnitt 40 fokussiert wird. Die Teilstrahl-Linse 48 übersetzt die laterale Position des ersten Fokuspunkts 46 in einen Strahlwinkel, der wiederum von der Objektivlinse 19 in eine laterale Position übersetzt wird. Durch Betätigen der Scanspiegel 27, 28 kann mit dem ersten spektralen Teilstrahl 32 also der vordere Augenabschnitt 40 gescannt werden, so dass eine Bildgebung des vorderen Augenabschnitts 40 möglich wird.

Werden der zweite dichroitischen Teilerspiegel 36, der zweite Teilstrahl-Spiegel 38 und die Objektivlinse 19 entlang der Strahlrichtung des zweiten Teil-Strahlengangs 33 gegenüber dem ersten spektralen Teilerspiegel 35 und dem ersten Teilstrahl-Spiegel 37 verschoben und dabei die Distanz zwischen der Objektivlinse 19 und dem Auge 14 aufrechterhalten, so ändert sich die Fokuslage des zweiten spektralen Teilstrahls 33 in Bezug auf den hinteren Augenabschnitt 41, während die Fokuslage des ersten spektralen Teilstrahls 32 in Bezug auf den vorderen Augenabschnitt 40 unverändert bleibt. Damit wird es möglich, auch bei fehlsichtigen Patienten den zweiten spektralen Teilstrahl 33 auf den hinteren Augenabschnitt 41 zu fokussieren.

Bei den bislang beschriebenen Ausführungsbeispielen sind die wellenlängenabhängigen Strahlenteiler als dichroitische Teilerspiegel 35, 36 ausgestaltet, so dass ein Teil der Frequenzen des OCT-Lichts 15 reflektiert wird und ein anderer Teil der Frequenzen des OCT-Lichts 15 transmittiert wird. Die Erfindung kann auch mit anderen Arten von wellenlängenabhängigen Strahlenteilern verwirklicht werden.

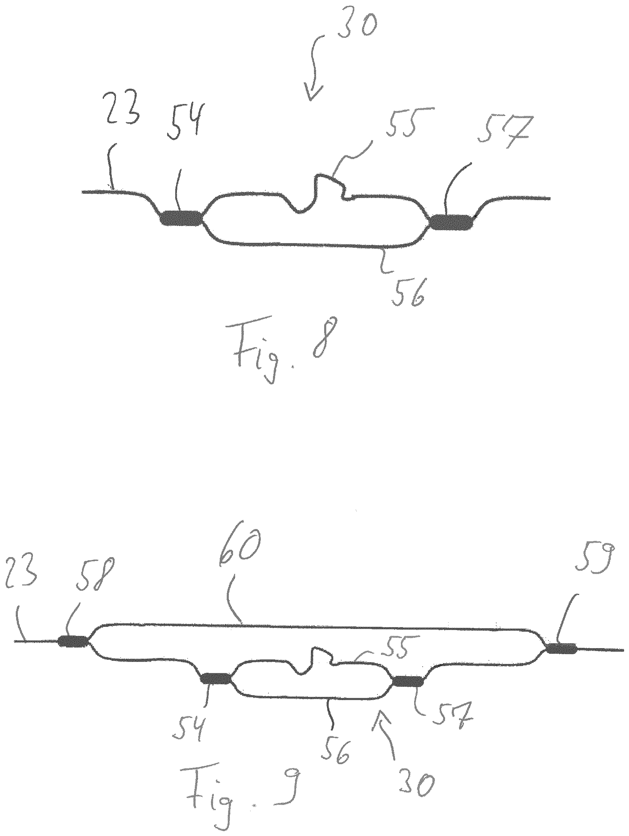

In Fig. 8 ist ein Ausführungsbeispiel dargestellt, bei dem der Parallelabschnitt 30 des Objektstrahlengangs 23 über Lichtleiter von verschiedener Länge realisiert ist. Das OCT-Licht des Objektstrahlengangs trifft auf einen Faserkoppler 54, von dem das OCT-Licht frequenzabhängig entweder in eine erste Faser 55 oder in eine zweite Faser 56 eingeleitet wird. Nachdem verschiedene lange Wegstrecken zurückgelegt wurden, werden der erste spektrale Teilstrahl 32 und der zweite spektrale Teilstrahl 33 in einem zweiten Faserkoppler 57 wieder zusammengeführt. Der erste Faserkoppler 54 entspricht in seiner Funktion dem ersten spektralen Teilerspiegel 35, der zweite Faserkoppler 57 entspricht in seiner Funktion dem zweiten spektralen Teilerspiegel 36. Genau wie bei allen zuvor beschriebenen Varianten kann der Parallelabschnitt 30 entweder im Objektstrahlengang 23 oder im Referenzstrahlengang 24 angeordnet sein.

Bei der weiteren Variante gemäß Fig. 9 ist eine dritte Faser 60 vorgesehen, mit der der Objektstrahlengang 23 an dem Parallelabschnitt 30 vorbeigeführt werden kann. Wird das gesamte Spektrum des OCT-Lichts auf einen Objektbereich geleitet, so ergibt sich eine Messung mit verbesserter axialer Auflösung. Das Umschalten zwischen dem Parallelabschnitt 30 und der dritten Faser 60 erfolgt über faseroptische Schaltelemente 54, 57, die ähnlich einer Weiche einen Pfad auf der einen Seite mit einem von zwei Pfaden auf der anderen Seite lichtleitend verbinden. Das Umschalten zwischen den zwei Pfaden erfolgt über ein digitales elektrisches Signal. Das Umschalten auf die dritte Faser 60 entspricht dem Ausklappen der Einheit 42 mit den dichroitischen Teilerspiegeln 35, 36 aus dem Strahlengang bei den zuvor beschriebenen Ausführungsbeispielen.

## Patentansprüche

1. OCT-System mit einer OCT-Lichtquelle (16) zum Aussenden von OCT-Licht (15) in einen Objektstrahlengang (23) und einen Referenzstrahlengang (24) und mit einem Detektor (25, 52, 53) zum Aufnehmen eines aus dem Objektstrahlengang (23) und dem Referenzstrahlengang (24) erzeugten Interferenzsignals, wobei das OCT-System einen in dem OCT-Strahlengang anordenbaren wellenlängenabhängigen Strahlenteiler (35, 36, 54, 57) umfasst, so dass ein erster spektraler Teilstrahl (32) entlang einer längeren Wegstrecke geleitet wird und dass ein zweiter spektraler Teilstrahl (33) entlang einer kürzere Wegstrecke geleitet wird, wobei die OCT-Lichtquelle (16) eine einheitliche OCT-Lichtquelle (16) ist, die das OCT-Licht (15) für den ersten spektralen Teilstrahl (32) und für den zweiten spektralen Teilstrahl (33) bereitstellt,
**dadurch gekennzeichnet, dass**
die OCT-Lichtquelle (16) mit einem einheitlichen Durchstimmvorgang den ersten spektralen Teilstrahl (32) und den zweiten spektralen Teilstrahl (33) erzeugt.

2. OCT-System nach Anspruch 1, **dadurch gekennzeichnet, dass** der OCT-Strahlengang einen Parallelabschnitt (30) umfasst, in dem der erste spektrale Teilstrahl (32) entlang einer ersten Parallelstrecke (66) geleitet wird und in dem der zweite spektrale Teilstrahl (33) entlang einer zweiten Parallelstrecke (67) geleitet wird.

3. OCT-System nach Anspruch 2, **dadurch gekennzeichnet, dass** ein erster wellenlängenabhängiger Strahlenteiler (35, 54) an einem Eingang des Parallelabschnitts (30) angeordnet ist und dass ein zweiter wellenlängenabhängiger Strahlenteiler (36, 57) an einem Ausgang des Parallelabschnitts (30) angeordnet ist.

4. OCT-System nach Anspruch 2 oder 3, **dadurch gekennzeichnet, dass** die Weglängendifferenz zwischen der ersten Parallelstrecke (66) und der zweiten Parallelstrecke (67) einstellbar ist.

5. OCT-System nach Anspruch 4, **dadurch gekennzeichnet, dass** der Abstand zwischen einem in dem Parallelabschnitt angeordneten Teilstrahl-Spiegel (37, 38) und dem wellenlängenabhängigen Strahlenteiler (35, 36) einstellbar ist.

6. OCT-System nach einem der Ansprüche 1 bis 5, **dadurch gekennzeichnet, dass** in einem ersten Zustand der wellenlängenabhängige Strahlenteiler (35, 36) in dem OCT-Strahlengang angeordnet ist und dass in einem zweiten Zustand der wellenlängenabhängige Strahlenteiler (35, 36) nicht in dem OCT-Strahlengang angeordnet ist.

7. OCT-System nach Anspruch 6, **gekennzeichnet durch** einen Aktor (61), mit dem der Abstand zwischen dem Teilstrahl-Spiegel (37, 38) und dem wellenlängenabhängigen Strahlenteiler (35, 36) eingestellt wird und mit dem zwischen dem ersten Zustand und dem zweiten Zustand des wellenlängenabhängigen Strahlenteilers (35, 36) gewechselt wird.

8. OCT-System nach einem der Ansprüche 1 bis 7, **dadurch gekennzeichnet, dass** die axiale Fokuslage des ersten spektralen Teilstrahls (32) von der axialen Fokuslage des zweiten spektralen Teilstrahls (33) abweicht.

9. OCT-System nach einem der Ansprüche 1 bis 8, **dadurch gekennzeichnet, dass** der erste spektrale Teilstrahl (32) unter einem anderen Winkel auf das Messobjekt (14) trifft als der zweite spektrale Teilstrahl (33).

10. OCT-System nach einem der Ansprüche 1 bis 9, **gekennzeichnet durch** eine in dem Parallelabschnitt (30) angeordnete Teilstrahl-Linse (43, 48).

11. OCT-System nach Anspruch 10, **dadurch gekennzeichnet, dass** die Teilstrahl-Linse (43, 48) verstellbar ist.

12. OCT-System nach einem der Ansprüche 2 bis 11, **gekennzeichnet durch** eine außerhalb des Parallelabschnitts (30) angeordnete Gesamtstrahl-Linse (45), die den ersten spektralen Teilstrahl (32) in einem ersten Fokuspunkt (46) innerhalb des Parallelabschnitts (30) fokussiert und die den zweiten spektralen Teilstrahl (33) in einem zweiten Fokuspunkt (47) innerhalb des Parallelabschnitts (30) fokussiert.

13. OCT-System nach Anspruch 12, **dadurch gekennzeichnet, dass** die Gesamtstrahl-Linse (45) zwischen einer Scaneinrichtung (27, 28) und dem Parallelabschnitt (30) angeordnet ist.

14. OCT-Verfahren, bei dem OCT-Licht (15) ausgesendet wird und in einen Objektstrahlengang (23) und einen Referenzstrahlengang (24) aufgespalten wird, wobei mit einem Detektor (25, 52, 53,) ein aus dem Objektstrahlengang (23) und dem Referenzstrahlengang (24) erzeugtes Interferenzsignal aufgenommen und wobei in dem Strahlengang des OCT-Lichts ein wellenlängenabhängiger Strahlenteiler (35, 36, 54, 57) angeordnet ist, so dass ein erster spektraler Teilstrahl (32) des OCT-Lichts (15) entlang einer längeren Wegstrecke geleitet wird und dass ein zweiter spektraler Teilstrahl (33) des OCT-Lichts (15) entlang einer kürzere Wegstrecke geleitet wird, wobei das OCT-Licht (15) für den ersten spektralen Teilstrahl (32) und für den zweiten spektralen Teilstrahl (33) mit einer einheitlichen OCT-Lichtquelle (16) bereitgestellt wird,
**dadurch gekennzeichnet, dass**
die OCT-Lichtquelle (16) mit einem einheitlichen Durchstimmvorgang den ersten spektralen Teilstrahl (32) und den zweiten spektralen Teilstrahl (33) erzeugt.

## Claims

1. An OCT system comprising an OCT light source (16) for emitting OCT light (15) into an object beam path (23) and a reference beam path (24) and comprising a detector (25, 52, 53) for picking up an interference signal generated from the object beam path (23) and the reference beam path (24), wherein the OCT system comprises a wavelength-dependent beam splitter (35, 36, 54, 57) arrangeable in the OCT beam path, such that a first spectral partial beam (32) is guided along a longer path distance and that a second spectral partial beam (33) is guided along a shorter path distance, wherein the OCT light source (16) is a unified OCT light source (16) which provides the OCT light (15) for the first spectral partial beam (32) and for the second spectral partial beam (33), **characterized in that** the OCT light source (16) generates the first spectral partial beam (32) and the second spectral partial beam (33) by means of a unified tuning process.

2. The OCT system as claimed in claim 1, **characterized in that** the OCT beam path comprises a parallel section (30), in which the first spectral partial beam (32) is guided along a first parallel path (66) and in which the second spectral partial beam (33) is guided along a second parallel path (67).

3. The OCT system as claimed in claim 2, **characterized in that** a first wavelength-dependent beam splitter (35, 54) is arranged at an input of the parallel section (30) and **in that** a second wavelength-dependent beam splitter (36, 57) is arranged at an output of the parallel section (30).

4. The OCT system as claimed in claim 2 or 3, **characterized in that** the path length difference between the first parallel path (66) and the second parallel path (67) is adjustable.

5. The OCT system as claimed in claim 4, **characterized in that** the distance between a partial beam mirror (37, 38) arranged in the parallel section and the wavelength-dependent beam splitter (35, 36) is adjustable.

6. The OCT system as claimed in any of claims 1 to 5, **characterized in that** in a first state the wavelength-dependent beam splitter (35, 36) is arranged in the OCT beam path and **in that** in a second state the wavelength-dependent beam splitter (35, 36) is not arranged in the OCT beam path.

7. The OCT system as claimed in claim 6, **characterized by** an actuator (61), which is used to adjust the distance between the partial beam mirror (37, 38) and the wavelength-dependent beam splitter (35, 36) and which is used to change between the first state and the second state of the wavelength-dependent beam splitter (35, 36).

8. The OCT system as claimed in any of claims 1 to 7, **characterized in that** the axial focus position of the first spectral partial beam (32) deviates from the axial focus position of the second spectral partial beam (33).

9. The OCT system as claimed in any of claims 1 to 8, **characterized in that** the first spectral partial beam (32) impinges on the measurement object (14) at a different angle than the second spectral partial beam (33).

10. The OCT system as claimed in any of claims 1 to 9, **characterized by** a partial beam lens (43, 48) arranged in the parallel section (30).

11. The OCT system as claimed in claim 10, **characterized in that** the partial beam lens (43, 48) is adjustable.

12. The OCT system as claimed in any of claims 2 to 11, **characterized by** a total beam lens (45) arranged outside the parallel section (30), which total beam lens focuses the first spectral partial beam (32) at a first focal point (46) within the parallel section (30) and focuses the second spectral partial beam (33) at a second focal point (47) within the parallel section (30).

13. The OCT system as claimed in claim 12, **characterized in that** the total beam lens (45) is arranged between a scanning device (27, 28) and the parallel section (30).

14. An OCT method in which OCT light (15) is emitted and is split into an object beam path (23) and a reference beam path (24), wherein an interference signal generated from the object beam path (23) and the reference beam path (24) is picked up by a detector (25, 52, 53) and wherein a wavelength-dependent beam splitter (35, 36, 54, 57) is arranged in the beam path of the OCT light, such that a first spectral partial beam (32) of the OCT light (15) is guided along a longer path distance and that a second spectral partial beam (33) of the OCT light (15) is guided along a shorter path distance, wherein the OCT light (15) for the first spectral partial beam (32) and for the second spectral partial beam (33) is provided by a unified OCT light source (16), **characterized in that** the OCT light source (16) generates the first spectral partial beam (32) and the second spectral partial beam (33) by means of a unified tuning process.

## Revendications

1. Système de tomographie par cohérence optique (OCT), comprenant une source de lumière OCT (16) servant à émettre de la lumière OCT (15) dans un trajet de faisceau d'objet (23) et un trajet de faisceau de référence (24), et comprenant un détecteur (25, 52, 53) servant à enregistrer un signal d'interférence produit à partir du trajet de faisceau d'objet (23) et du trajet de faisceau de référence (24), le système OCT comprenant un séparateur de faisceau (35, 36, 54, 57) dépendant de la longueur d'onde et pouvant être disposé sur le trajet de faisceau OCT de sorte qu'un premier faisceau partiel spectral (32) est guidé le long d'un parcours plus long et qu'un deuxième faisceau partiel spectral (33) est guidé le long d'un parcours plus court, la source de lumière OCT (16) étant une source de lumière OCT homogène (16) qui fournit la lumière OCT (15) pour le premier faisceau partiel spectral (32) et pour le deuxième faisceau partiel spectral (33),
**caractérisé en ce que** la source de lumière OCT (16) produit le premier faisceau partiel spectral (32) et le deuxième faisceau partiel spectral (33) par un processus d'accord homogène.

2. Système OCT selon la revendication 1, **caractérisé en ce que** le trajet de faisceau OCT comprend une section parallèle (30) dans laquelle le premier faisceau partiel spectral (32) est guidé le long d'une première voie parallèle (66) et dans laquelle le deuxième faisceau partiel spectral (33) est guidé le long d'une deuxième voie parallèle (67).

3. Système OCT selon la revendication 2, **caractérisé en ce qu'**un premier séparateur de faisceau (35, 54) dépendant de la longueur d'onde est disposé à une entrée de la section parallèle (30), et **en ce qu'**un deuxième séparateur de faisceau (36, 57) dépendant de la longueur d'onde est disposé à une sortie de la section parallèle (30) .

4. Système OCT selon la revendication 2 ou 3, **caractérisé en ce que** la différence de longueur de chemin entre la première voie parallèle (66) et la deuxième voie parallèle (67) est réglable.

5. Système OCT selon la revendication 4, **caractérisé en ce que** la distance entre un miroir de faisceau partiel (37, 38) disposé dans la section parallèle et le séparateur de faisceau (35, 36) dépendant de la longueur d'onde est réglable.

6. Système OCT selon l'une quelconque des revendications 1 à 5, **caractérisé en ce que** dans un premier état, le séparateur de faisceau (35, 36) dépendant de la longueur d'onde est disposé sur le trajet de faisceau OCT, et **en ce que** dans un deuxième état, le séparateur de faisceau (35, 36) dépendant de la longueur d'onde n'est pas disposé sur le trajet de faisceau OCT.

7. Système OCT selon la revendication 6, **caractérisé par** un actionneur (61) qui permet de régler la distance entre le miroir de faisceau partiel (37, 38) et le séparateur de faisceau (35, 36) dépendant de la longueur d'onde et d'alterner entre le premier état et le deuxième état du séparateur de faisceau (35, 36) dépendant de la longueur d'onde.

8. Système OCT selon l'une quelconque des revendications 1 à 7, **caractérisé en ce que** la position focale axiale du premier faisceau partiel spectral (32) est différente de la position focale axiale du deuxième faisceau partiel spectral (33).

9. Système OCT selon l'une quelconque des revendications 1 à 8, **caractérisé en ce que** le premier faisceau partiel spectral (32) est incident sur l'objet de mesure (14) sous un autre angle que le deuxième faisceau partiel spectral (33).

10. Système OCT selon l'une quelconque des revendications 1 à 9, **caractérisé par** une lentille de faisceau partiel (43, 48) disposée dans la section parallèle (30).

11. Système OCT selon la revendication 10, **caractérisé en ce que** la lentille de faisceau partiel (43, 48) est réglable.

12. Système OCT selon l'une quelconque des revendications 2 à 11, **caractérisé en ce que** par une lentille de faisceau total (45) disposée en dehors de la section parallèle (30) et qui focalise le premier faisceau partiel spectral (32) en un premier point de focalisation (46) à l'intérieur de la section parallèle (30) et qui focalise le deuxième faisceau partiel spectral (33) en un deuxième point de focalisation (47) à l'intérieur de la section parallèle (30).

13. Système OCT selon la revendication 12, **caractérisé en ce que** la lentille de faisceau total (45) est disposée entre un dispositif de balayage (27, 28) et la section parallèle (30).

14. Procédé OCT, dans lequel la lumière OCT (15) est émise et divisée en un trajet de faisceau d'objet (23) et un trajet de faisceau de référence (24), un détecteur (25, 52, 53) enregistrant un signal d'interférence produit à partir du trajet de faisceau d'objet (23) et du trajet de faisceau de référence (24), et un séparateur de faisceau (35, 36, 54, 57) dépendant de la longueur d'onde étant disposé sur le trajet de faisceau de la lumière OCT de sorte qu'un premier faisceau partiel spectral (32) de la lumière OCT (15) est guidé le long d'un parcours plus long et qu'un deuxième faisceau partiel spectral (33) de la lumière OCT (15) est guidé le long d'un parcours plus court, la lumière OCT (15) pour le premier faisceau partiel spectral (32) et pour le deuxième faisceau partiel spectral (33) étant fournie par une source de lumière OCT homogène (16),
**caractérisé en ce que** la source de lumière OCT (16) produit le premier faisceau partiel spectral (32) et le deuxième faisceau partiel spectral (33) par un processus d'accord homogène.
